# EUROPEAN PATENT APPLICATION

(11) **EP 4 397 745 A1**
(43) Date of publication of application: **10.07.2024**
(21) Application number: 23157898.0
(22) Date of filing: 22.02.2023
(51) Int. Cl.: C12M 1/26

(54) **CELL SHEET AND PREPARATION METHOD THEREOF**

(30) Priority: 09.01.2023 TW 112100873
(71) Applicant: Up Cell Biomedical Inc., New Taipei City 221416 (TW)
(72) Inventor: LIN, Hong-You, 221416 New Taipei City (TW); HSIEH, Kai-Ju, 221416 New Taipei City (TW); LIN, Jia-Hui, 221416 New Taipei City (TW); CHEN, Tsung-Chi, 221416 New Taipei City (TW)
(74) Representative: Becker, Eberhard

(57) **Abstract**

The invention provides a cell sheet and a preparation method thereof, and the preparation method includes the following steps. Cells are grown in a cell culture dish to form a cell sheet. Afterwards, a culture supernatant in the cell culture dish is removed, physiological saline is added to cover the cell sheet, and then the cell culture dish is placed on an ultrasonic oscillator for physical vibration, so that the cell sheet and the cell culture dish are separated. Next, the cell sheet is obtained by using a cell-adhesive paper or rinse.

## Description

### BACKGROUND

### Technical Field

The disclosure relates to a cell sheet and a preparation method thereof.

### Description of Related Art

Due to the advancement of medical engineering, the medical treatment of human injuries has undergone great changes. A new tissue engineering "cell sheet" has been developed, which is made of whole cells without adding any support.

Existing market technology is to utilize the special coating on the petri dish to change the configuration of the coating through temperature changes, so that the cell sheet cultivated on it can be naturally removed from the plate by temperature changes. However, it is limited by temperature and time requirements. It is necessary to wait the temperature sensing plate for 20 minutes to 30 minutes in an environment of 20°C. Depending on the type of cells, the time to take off the plate is also different, and it is possible to wait for 20 minutes to 60 minutes, or even longer, the difference is huge, so it cannot be used immediately in the back-end medical treatment. During the cultivation process, extra attention should be paid to temperature control. The temperature-sensitive culture dish needs to be placed on a temperature-controlled machine for operation during the whole process, which is troublesome in terms of manufacturing process. In addition, the cost of special temperature-sensitive plates is relatively high, and there may be problems such as shortages.

Based on the above, developing a cell sheet and a preparation method thereof, which can effectively reduce the overall production cost and time cost, and improve the problem of immediate application on the back-end medical treatment and the shortcoming of troublesome manufacturing process, is an important subject of current research.

### SUMMARY

The invention provides a cell sheet and a preparation method thereof, which can effectively reduce the overall production cost and time cost, and improve the problem of instant application in back-end medical treatment and the disadvantages of troublesome manufacturing process.

The preparation method of the invention includes the following steps. Cells are grown in a cell culture dish to form a cell sheet. Afterwards, a culture supernatant in the cell culture dish is removed, physiological saline is added to cover the cell sheet, and then the cell culture dish is placed on an ultrasonic oscillator for physical vibration, so that the cell sheet and the cell culture dish are separated. Next, the cell sheet is obtained by using a cell-adhesive paper or rinse.

In an embodiment of the invention, the cell sheet is separated from the cell culture dish at a temperature of 20°C to 37°C.

In an embodiment of the invention, a time for growing the cells in the cell culture dish is 2 days to 5 days.

In an embodiment of the invention, a power of placing the cell culture dish on the ultrasonic oscillator for the physical vibration is 10 watts to 50 watts, and an oscillation time is 30 seconds to 5 minutes.

In an embodiment of the invention, when a size of the cell sheet is greater than 1 cm in diameter, the cell-adhesive paper is used to obtain the cell sheet, and the cell-adhesive paper is used to adhere and support the cell sheet from the cell culture dish, the cell-adhesive paper includes a glassine paper, a baking paper, a parchment paper, a kraft paper, a scale paper, a lining paper, a pearlescent paper or a greaseproof paper.

In an embodiment of the invention, when a size of the cell sheet is less than 1 cm in diameter, rinse to obtain the cell sheet

A cell sheet of the invention is prepared by using the above-mentioned preparation method.

In an embodiment of the invention, a delivery condition of the cell sheet is refrigerated temperature 4°C to 20°C.

In an embodiment of the invention, a thickness of the cell sheet is 0.1 µm to 70 µm.

In an embodiment of the invention, the cell sheet has a surface area of 0.01 cm² to 30 cm².

Based on the above, the invention provides a cell sheet and a preparation method thereof, wherein the thermosensitive dish in the prior art is not used, but physical vibration (ultrasonic vibration) principle is utilized to accelerate the cell sheet to be removed from the cell culture dish, and the cell viability is not affected. In this way, a complete cell sheet can be obtained in a very short time, and it can be used in any petri dish and suitable for any size of cell sheet. With the cell sheet and the preparation method thereof of the invention, primary cells from patients can be cultured into cell sheets of different sizes, and transplanted back to the diseased site of the patients to promote tissue regeneration and repair, thereby improving the patient's quality of life (QOL).

### DESCRIPTION OF THE EMBODIMENTS

Embodiments of the disclosure will be described in details below. However, these embodiments are illustrative, and the disclosure is not limited thereto.

Herein, a range indicated by "one value to another value" is a general representation which avoids enumerating all values in the range in the specification. Therefore, the description of a specific numerical range covers any numerical value within the numerical range and the smaller numerical range bounded by any numerical value within the numerical range, as if the arbitrary numerical value and the smaller numerical range are written in the specification.

The preparation method of the invention includes the following steps. Cells are grown in a cell culture dish to form a cell sheet. Afterwards, a culture supernatant in the cell culture dish is removed, physiological saline is added to cover the cell sheet, and then the cell culture dish is placed on an ultrasonic oscillator for physical vibration, so that the cell sheet and the cell culture dish are separated. Next, the cell sheet is obtained by using a cell-adhesive paper or rinse.

In the present embodiment, a time to grow cells in the cell culture dish is about 2 days to 5 days. The temperature sensing dish in the prior art is not necessary, so the cells can be grown in any petri dish. Therefore, the long cultivation time of the temperature sensing disc in the prior art which is 14 days can be shortened, cost can be reduced, and possible problem of the temperature sensor plate which might be out of stock can be avoided. The cell culture dish is placed on the ultrasonic oscillator for physical vibration, for example, with a power of 10 watts to 50 watts, and a vibration time of, for example, 30 seconds to 5 minutes. By placing the cell culture dish on the ultrasonic oscillator for physical vibration, the cell sheet and the cell culture dish are separated at a temperature of 20°C to 37°C, for example, that is, the cell sheet and the cell culture dish can be separated at room temperature. In this way, different from the waiting time of 20 minutes to 30 minutes in an environment of 20°C in the prior art, the invention is not limited by temperature or time requirements.

In the present embodiment, when the size of the cell sheet is greater than 1 cm in diameter, the cell-adhesive paper is used to obtain the cell sheet; when the size of the cell sheet is less than 1 cm in diameter, rinse to obtain the cell sheet. In this way, the overall harvesting process can be completed in only 5 to 10 minutes. Compared with the existing technology in the market, the invention can greatly shorten the cell sheet production time and process cost, and the process is simpler and more convenient. In more detail, the cell-adhesive paper can be any material that can adhere to and support the cell sheet from the cell culture dish, including but not limited to a glassine paper, a baking paper, a parchment paper, a kraft paper, a scale paper, a lining paper, a pearlescent paper or a greaseproof paper etc.

The invention also proposes a cell sheet, which is prepared by using the above-mentioned preparation method. In the present embodiment, the thickness of the cell sheet is, for example, 0.1 µm to 70 µm, and the surface area of the cell sheet is, for example, 0.01 cm² to 30 square cm². Compared with the existing market technology, with the preparation method of cell sheet of the invention, thicker cell sheets with more cells can be obtained from different sources of cell culture. Considering that the current clinically used cell sheets are thin, it is necessary to stack several cell sheets to facilitate treatment. If the cell sheet of the invention is applied to clinical treatment, it is expected to reduce the number of cell sheets required, and it may also be used directly without stacking. As a result, the difficulties of back-end medical applications can be improved, and the original cell therapy effect can be maintained. In addition, since the cell sheet of the invention is thicker, the cell sheet of the invention is less likely to be damaged compared to the thinner cell sheet of the prior art. On the other hand, with the preparation method of cell sheet of the invention, the cell sheets of any size can be manufactured according to actual needs, such as an injectiontype sheet and the like.

The shipping condition of the cell sheet of the invention is, for example, a refrigerated temperature of 4°C to 20°C. Therefore, compared to the cell sheet of the existing market technology, which needs to be shipped at a temperature of 37°C, the cell sheet of the invention can be transported through refrigerated temperature for proper storage. In addition, the cell sheet of the invention can be used directly after the preparation is completed. Compared with the cell sheet of the existing market technology, which needs to wait for 20 minutes to separate the cell sheet before use, the cell sheet of the invention can effectively reduce the time cost and improve the back-end difficulties in medical application, but also has practical applicability.

In summary, the invention provides a cell sheet and a preparation method thereof, mainly by placing the cell culture dish on an ultrasonic oscillator for physical vibration, so as to separate the cell sheet from the cell culture dish. Since there is no need to use the temperature-sensitive plate of the existing market technology, it is not limited by the temperature and time requirements, and can effectively reduce the overall manufacturing cost and time cost, improve the disadvantages of troublesome manufacturing process, and avoid the possible shortage problem, which is suitable for real-time application in back-end medical treatment. The invention can obtain thicker cell sheets with more cells from different sources of cell culture. In terms of clinical application, it is expected to reduce the number of cell sheets required, and it may also be used directly without stacking, which solves the difficulty of back-end medical applications, and maintains the original cell therapy effect with substantial applicability. The cell sheet of the invention can be applied to stroke treatment, oral cavity treatment, esophageal repair, spinal injury treatment, joint repair, skin damage repair, smoothing of wrinkles, repair of other organs, etc. However, the invention is not limited thereto, and the clinical applications mentioned here are only illustrative examples, and can also be applied to other disease treatments or clinical applications according to actual needs.

## Claims

1. A preparation method of a cell sheet, comprising:
growing cells in a cell culture dish to form a cell sheet;
removing a culture supernatant from the cell culture dish, adding physiological saline to cover the cell sheet, and then placing the cell culture dish on an ultrasonic oscillator for physical vibration, so that the cell sheet and the cell culture dish are separated; and
obtaining the cell sheet by using a cell-adhesive paper or rinse.

2. The preparation method of claim 1, wherein the cell sheet is separated from the cell culture dish at a temperature of 20°C to 37°C.

3. The preparation method of claim 1, wherein a time for growing the cells in the cell culture dish is 2 days to 5 days.

4. The preparation method of claim 1, wherein a power of placing the cell culture dish on the ultrasonic oscillator for the physical vibration is 10 watts to 50 watts, and an oscillation time is 30 seconds to 5 minutes.

5. The preparation method of claim 1, wherein when a size of the cell sheet is greater than 1 cm in diameter, the cell-adhesive paper is used to obtain the cell sheet, and the cell-adhesive paper is used to adhere and support the cell sheet from the cell culture dish, the cell-adhesive paper includes a glassine paper, a baking paper, a parchment paper, a kraft paper, a scale paper, a lining paper, a pearlescent paper or a greaseproof paper.

6. The preparation method of claim 1, wherein when a size of the cell sheet is less than 1 cm in diameter, rinse to obtain the cell sheet

7. A cell sheet, prepared by using the preparation method of any one of claims 1 to 6.

8. The cell sheet of claim 7, wherein a delivery condition of the cell sheet is refrigerated temperature 4°C to 20°C.

9. The cell sheet of claim 7, wherein a thickness of the cell sheet is 0.1 µm to 70 µm.

10. The cell sheet of claim 7, wherein the cell sheet has a surface area of 0.01 cm² to 30 cm².
